# EUROPÄISCHE PATENTANMELDUNG

(11) **EP 2 522 333 A2**
(43) Veröffentlichungstag der Anmeldung: **14.11.2012**
(21) Anmeldenummer: 12156135.1
(22) Anmeldetag: 20.02.2012
(51) Int. Cl.: A61K 8/365, A61Q 1/08, A61Q 1/10, A61K 8/02

(54) **Kosmetisches Puderprodukt**

(30) Priorität: 14.03.2011 DE 102011001256
(71) Anmelder: BCM Cosmetic GmbH, 63128 Dietzenbach (DE)
(72) Erfinder: Schröder, Katrin, 63128 Dietzenbach (DE); Manderla, Simone, 63128 Dietzenbach (DE)
(74) Vertreter: Meyer-Dulheuer, Karl-Hermann

(57) **Zusammenfassung**

Die vorliegende Erfindung betrifft ein Puderprodukt, welches nach dem Auftragen auf die Haut eine andere Farbe entwickelt, als die Farbe des Puderprodukts vor dem Applizieren auf die Haut aufweist. Hierzu wird ein kosmetisches Puderprodukt sowie ein Herstellungsverfahren für ein solches Puderprodukt vorgeschlagen, wobei das Puderprodukt mindestens einen kosmetischen Farbstoff enthält, dessen Färbung abhängig von pH-Wert und Polarität ist, wobei alle pulverförmigen Puderbestandteile des kosmetischen Puderproduktes mit einer Säure benetzt sind und der pH-Wert des kosmetischen Puderproduktes unter dem durchschnittlichen pH-Wert der Haut von pH 5,5 liegt.

## Beschreibung

Die vorliegende Erfindung betrifft ein Puderprodukt, welches nach dem Auftragen auf die Haut eine andere Farbe entwickelt, als die Farbe des Puderprodukts vor dem Applizieren auf die Haut aufweist. Hierzu wird ein kosmetisches Puderprodukt sowie ein Herstellungsverfahren für ein solches Puderprodukt vorgeschlagen, wobei das Puderprodukt mindestens einen kosmetischen Farbstoff enthält, dessen Färbung abhängig von pH-Wert und Polarität ist, wobei alle pulverförmigen Puderbestandteile des kosmetischen Puderproduktes mit einer Säure benetzt sind und der pH-Wert des kosmetischen Puderproduktes unter dem durchschnittlichen pH-Wert der Haut von pH 5,5 liegt.

Ziel der vorliegenden Erfindung war es, ein kosmetisches Puderprodukt bereitzustellen, welches nach dem Auftragen auf die Haut eine andere Farbe entwickelt, als die Farbe des Produkts vor dem Auftragen. Hierzu sollen kosmetische Farbstoffe eingesetzt werden, dessen Färbung abhängig von pH-Wert und Polarität ist. Die verwendeten Farbstoffe sollen die Eigenschaft haben, dass sie bei einem pH-Wert unter dem durchschnittlichen pH-Wert der Haut eine andere Färbung aufweisen als nach dem Auftragen auf die Haut. Dadurch soll nach dem Applizieren auf die Haut ein deutlicher Farbwechsel vollzogen werden.

Die meisten Puderbestandteile, wie zum Beispiel Talkum sind jedoch hygroskopisch und haben somit einen gewissen Anteil an Feuchtigkeit. In Kontakt mit kosmetischen Farbstoffen, dessen Färbung abhängig von pH-Wert und Polarität ist, erfolgt meist eine Farbveränderung der Puderbestandteile während der Herstellung des Puderproduktes. Dieser Effekt ist nur bedingt reversibel. Der Farbwechsel der Farbstoffe ist abhängig von der Polarität sowie des umgebenden pH-Wertes. Bei einem niedrigen pH-Wert sowie auch bei Kontakt mit nicht polaren oder wenig polaren Bestandteilen tritt nämlich keine Verfärbung auf.

Bisher sind nur wenige Produkte aus dem Stand der Technik bekannt, welche einen vergleichbaren Effekt erzielen sollen. Ein Puderprodukt in Form von Rouge lobt einen vergleichbaren Effekt aus. Bei diesem Rouge soll sich die Farbe des Produktes nach dem Auftragen auf die Haut deutlich intensivieren. Dies soll den Effekt nachahmen, dass die Person, die das Produkt aufträgt "errötet". Allerdings weist dieses Produkt bereits vor dem Auftragen eine deutliche Färbung auf. Der Bulk hat bereits eine rosafarbene Färbung, welche sich nach dem Auftragen auf die Haut nicht wesentlich verändert. In diesem Fall wird lediglich eine leichte intensivere Färbung auf der Haut erreicht, jedoch im selben Farbton, wie die bereits im Bulk vorhandene Farbe. Bei diesem Produkt ist somit kein wirklicher Farbwechsel oder Veränderung des Farbtons nach dem Applizieren auf die Haut zu erkennen, da der Farbstoff bereits im Bulk seine Färbung ausgebildet hat. Dies soll jedoch vorliegend verhindert werden. In diesem Produkt aus dem Stand der Technik ist der Farbstoff mit der *Colour Index (CI) Constitution Number* CI 45410:1 enthalten und das Puderprodukt weist einen pH-Wert von pH 6,9 auf, bei welchem der Farbstoff bereits eine rosa Färbung aufweist.

Zur Bestimmung des pH-Wertes eines Puderproduktes wird jeweils eine 10%-ige Dispersion in Wasser hergestellt und der pH-Wert bei Raumtemperatur und unter Normaldruck bestimmt.

Ein weiterer Nachteil der bereits im Stand der Technik eingesetzten Farbstoffe (Fluorescein-Derivate) ist die Tatsache, dass diese Farbstoffe nicht UV-stabil sind. Nach längerer Bestrahlung mit UV-Licht bleichen diese Farbstoffe häufig aus und es ist kein Farbwechsel-Effekt mehr zu erzielen. Dies ist insbesondere bei Kosmetikprodukten nicht erwünscht. Die vorliegende Erfindung soll demnach ebenfalls die bekannten Nachteile aus dem Stand der Technik überwinden.

Die Aufgabe der vorliegenden Erfindung wird durch ein kosmetisches Puderprodukt sowie durch ein Verfahren zur Herstellung eines solchen Puderprodukts gelöst, wobei das kosmetische Puderprodukt mindestens einen kosmetischen Farbstoff enthält, dessen Färbung abhängig von pH-Wert und Polarität ist, wobei alle Puderbestandteile des kosmetischen Puderproduktes mit einer Säure benetzt sind und der pH-Wert des kosmetischen Puderproduktes unter dem durchschnittlichen pH-Wert der Haut von pH 5,5 liegt.

Durch die gleichmäßige Benetzung aller pulverförmigen Puderbestandteile mit einer Säure wird der pH-Wert im Puderprodukt abgesenkt. Erst nach Absenkung des pH-Wertes wird der Farbstoff hinzugegeben, damit dieser in der Zubereitung farblos bleibt. Das erfindungsgemäße Puderprodukt weist somit aufgrund des reduzierten pH-Wertes keine Färbung auf, welche durch diesen Farbstoff verursacht wird. Wenn beispielsweise Talk als Füllstoff eingesetzt wird, weist das Produkt eine weiße Farbe auf. Je nach der gewünschten Färbung des Puderproduktes, können unterschiedliche Pigmente zugesetzt werden, die einen bestimmten Farbeindruck im Endprodukt hervorrufen können. Erst nach dem Applizieren des Puderprodukts auf die Haut ändert sich die Färbung des Farbstoffes aufgrund des pH-Wertes der Haut von etwa pH 5,5 und der Feuchtigkeit in der Haut. Bereits kurze Zeit nach dem Applizieren verfärbt sich das Puderprodukt auf der Haut und ein Farbwechsel erfolgt. Durch den Einsatz eines löslichen Farbstoffes wird eine besonders abriebfeste und langanhaltende Färbung der Haut erreicht, da dieser nicht wie Pigmente leicht von der Haut abgewischt werden kann. Der lösliche Farbstoff benetzt die oberste Hautschicht und ermöglicht somit eine effektive Einfärbung der Haut. Das Produkt weist eine sehr gute UV-Beständigkeit auch nach 24h Bestrahlung mit UV-Licht auf.

In einer bevorzugten Ausführungsform der Erfindung handelt es sich bei der Säure um mindestens eine der folgenden: Anorganische Säuren wie Halogenwasserstoffsäuren oder Sauerstoffsäuren, Organische Säuren wie Carbonsäuren (aliphatische oder aromatische Carbonsäuren), Fettsäuren oder Aminosäuren. Es können beispielsweise Stearinsäure, Zitronensäure oder Ascorbinsäure eingesetzt werden.

Im Sinne der vorliegenden Erfindung werden als Säuren neben den oben genannten Substanzen ebenfalls Substanzen bezeichnet, welche in Wasser gelöst einen pH-Wert von unter pH 5,5 erzielen.

In einer weiteren vorteilhaften Ausgestaltung der vorliegenden Erfindung liegt der pH-Wert des kosmetischen Puderproduktes unter pH 5,0, vorzugsweise unter pH 4,5. Um den Farbwechsel-Effekt bei Applikation auf die Haut zu erzielen, muss das Puderprodukt einen pH-Wert aufweisen, der unter dem pH-Wert liegt, bei dem die eingesetzten Farbstoffe ihren Farb-Umschlagspunkt haben. Bei den vorwiegend eingesetzten Farbstoffen hat sich ein pH-Wert zwischen pH 3,6 und pH 4,1 im Puderprodukt als besonders vorteilhaft herausgestellt.

In einer weiteren alternativen Ausführungsform weist der kosmetische Farbstoff innerhalb des Puderproduktes keine Färbung auf, wobei es sich bei dem kosmetischen Farbstoff vorzugsweise um einen Xanthen-Farbstoff, besonders bevorzugt um ein Fluorescein-Derivat oder ein Rhodamin-Derivat handelt.

Der kosmetische Farbstoff kann je nach pH-Wert in der neutralen, einfach negativ geladenen oder zweifach negativ geladenen Form, sowie ebenfalls als Natrium- oder Kaliumsalz vorliegen. Die Grundstruktur der Fluorescein-Derivate ist in der allgemeinen Strukturformel (1) in der offenen Carbonsäure-Form als Natriumsalz und in Strukturformel (11) in der Spiro-Lacton-Form dargestellt, wobei R kann bei einer einzelnen Verbindung mit R= H, Cl, Br, I oder NO₂ substituiert sein.

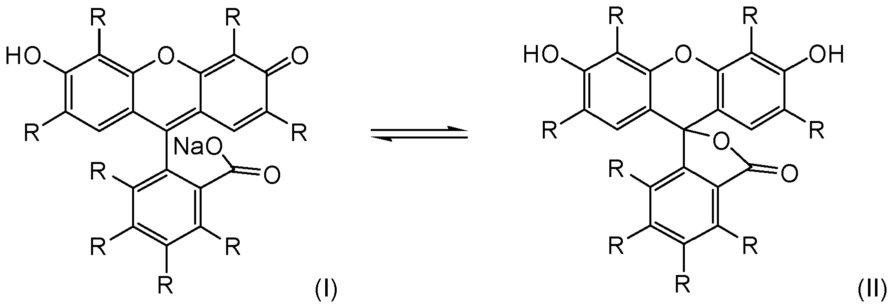

Diese Gruppe an Farbstoffen ermöglicht den erfindungsgemäßen Farbwechsel-Effekt. Als bevorzugte Farbstoffe für das erfindungsgemäße Puderprodukt haben sich Farbstoffe mit den *CI Constitution Numbers* CI 45350, CI 45370, CI 45380, CI 45396, CI 45405, CI 45410, CI 45430 erwiesen. Es kann erfindungsgemäß ebenfalls bevorzugt sein, Mischungen dieser Farbstoffe einzusetzen.

Die Farbstoffe CI 45370, CI 45380 und CI 45410 weisen im pH-Bereich unter pH 5,5 keine sichtbare Färbung auf. Nach dem Auftragen auf die Haut und somit Erreichen eines pH-Wertes von etwa pH 5,5 und entsprechender Feuchtigkeit, färben diese Farbstoffe das Puderprodukt vollständig und damit die Haut ein.

In einer vorteilhaften Ausgestaltungsform weist das Puderprodukt mindestens eine der folgenden pulverförmigen Komponenten auf: Füllstoffe, Pigmente, Perlmutt und Mischungen derselben.

Je nach Bedarf können der Zubereitung Füllstoffe zugesetzt werden. Füllstoffe haben einen positiven Einfluss auf die Textur und können die Produkteigenschaften hinsichtlich Feinheit, Gleichmäßigkeit, Haptik, Haltbarkeit, Natural-Finish und Sebumresistenz verändern. Außerdem können Füllstoffe visuelle Effekte im Endprodukt ermöglichen.

Als Füllstoffen können anorganische Substanzen, vorzugsweise Talk, Kaolin, Bornitrid, Silikate, insbesondere Zeolithe oder Schichtsilikate (Glimmer) eingesetzt werden. Bei den Schichtsilikaten ist die Verwendung von Muskovit, Phlogopit, Biotit, Serizit, Lepidolith, Paragonit, synthetischen Schichtsilikaten oder Mischungen derselben bevorzugt. Zudem können Zinkoxid, Titanoxid, Zirkoniumoxid, Ceroxid, Magnesiumcarbonat und Magnesiumhydrogencarbonat, Calciumcarbonat, Apatit-(CaOH), Glas oder Keramik-Mikrokapseln eingesetzt werden. An synthetischen organischen Polymeren können vorzugsweise Polycarbonate, Polyether, Polyester, Polyethylene, Polypropylen, Polyvinylchlorid, Polystyrole, Polyamide, Polyurethane, Polyacrylate, Poly-β-Alanin-Pulver oder Lauryllysin verwendet werden. Weitere bevorzugte organische Füllstoffe können ausgewählt werden aus Polytetrafluorethylen, Polymethylmethacrylaten, Silikonharzen oder Silikonelastomeren, Cellulosen, Stärken oder Stärkederivaten.

Bei gefärbten Produkten wie beispielsweise Lidschatten oder Rouge können eine Vielzahl unterschiedlicher Farbstoffe, Farbpigmente, Perlglanzpigmente oder Effektpigmente zusätzlich zu den erfindungsgemäßen Farbstoffen eingesetzt werden. Diese können sowohl einzeln als auch in einem Gemisch vorliegen sowie gegenseitig miteinander beschichtet sein, um diverse Farbeffekte zu erzielen. Die Partikel können unterschiedlich geformt sein, wie beispielsweise kugelig, oval, plättchenförmig oder ungleichmäßig geformt, sowie in beliebigen Kombinationen der verschiedenen Formen.
- An lipophilen Farbstoffen können beispielsweise Sudan®-Farbstoffe, D&C Red 17, D&C Green 6, Beta-Carotin, Sojaöl, D&C Yellow 11, D&C Violet 2, Chinolingelb und Annatto verwendet werden.
- Farbpigmente können aus weißen oder farbigen Pigmenten, anorganischen oder organischen Pigmenten, sowie beschichteten oder unbeschichteten Pigmenten ausgewählt werden. An anorganischen Pigmenten ist die Verwendung von Titandioxid, gegebenenfalls auch beschichtet, Zirkoniumoxid, Zinkoxid, Ceroxid, gelbem, rotem oder schwarzem Eisenoxid, natürlichen Aluminiumsilicaten wie Ocker, Glimmer und Kaolin, manganhaltigen Tonen wie Umbra und roter Bolus, Manganviolett, Ultramarin, Chromoxidgrün, Chromoxidhydratgrün, Chromhydroxid und Berliner Blau bevorzugt. An organischen Pigmenten können Ruß, FD&C Red 40, FD&C Yellow 5, FD&C Blue 1, D&C Green 5, Carmin-Lacke (aus Cochenille), sowie Verlackungen organischer Farbstoffe mit Aluminium, Barium, Calcium, Strontium, Zirkonium oder Mischungen der genannten Stoffe eingesetzt werden.
- Um besondere Farbeffekte zu erzielen, können außerdem verschiedene natürliche Perlglanzpigmente wie beispielweise "Fischsilber" (Guanin/Hypoxanthin-Mischkristalle aus Fischschuppen) oder "Perlmutt" (gemahlene Muschelschalen) eingesetzt werden. Perlglanzpigmente können weiterhin aus den weißen Perlglanzpigmenten ausgewählt werden, wie beispielsweise mit Titandioxid oder mit Bismutchloridoxid (BiOCl) beschichtete Glimmer.
- Außerdem können farbige Perlglanzpigmente wie Titandioxid-Glimmerpigmente mit Eisenoxiden, Titandioxid-Glimmerpigmente insbesondere mit Berliner Blau oder Chromoxid und Titandioxid-Glimmerpigmente mit unterschiedlichen organischen Pigmenten, sowie monokristalline Perlglanzpigmente wie zum Beispiel Bismutchloridoxid eingesetzt werden. Des Weiteren können auch plättchenförmige Metallpulver aus Aluminium, Bronze, Messing, Kupfer, Silber oder Gold verwendet werden. Diese Aufzählungen sind nur beispielhaft und keinesfalls abschließend.

Als pulverförmige Komponenten können im Puderprodukt sowohl hydrophobe als auch hydrophile pulverförmige Komponenten oder Mischungen derselben eingesetzt werden. Bei der hydrophoben pulverförmigen Komponente kann es sich um mindestens eine der folgenden handeln:
Talk, hydrophobe Polymer-Pulver, vorzugsweise Polyamide, besonders bevorzugt Polyamid 6.6, Polyethylen-Pulver, Polyfluor-Pulver, vorzugsweise Tetrafluorethylen-Polymere, Silikon-Pulver oder Polystyrol-Pulver. Als hydrophobe pulverförmige Komponenten eignen sich zudem Lipo-Aminosäuren, wie beispielsweise Lauryllysin und Bornitride.

Hydrophobe pulverförmige Komponenten können ebenfalls aus hydrophoben oder hydrophilen pulverförmigen Komponenten ausgewählt werden, die hydrophob beschichtet oder behandelt wurden. Die hydrophob beschichteten pulverförmigen Komponenten können vorzugsweise mit einem Silikon beschichtet sein. Pulverförmige Komponenten können ebenfalls mit Lecithinen oder pflanzlichen Wachsen, wie Carnaubawachs beschichtet werden. Ebenfalls möglich ist die Verwendung von Aminosäuren, Fluor-Derivaten, Mineralölen, Polyethylen, Polyacrylaten und/ oder Mischungen derselben. Bei hydrophob beschichteten und/oder behandelten Bestandteilen ist die Verwendung der folgenden pulverförmigen Komponenten bevorzugt: hydrophob beschichtete und/oder behandelte Glimmer, Silikate, Kaolin, Metalloxide, vorzugsweise Titandioxid, Eisenoxide, Zinkoxide und/oder Mischungen derselben.

In einer weiteren vorteilhaften Ausgestaltung handelt es sich bei der hydrophilen pulverförmigen Komponente im kosmetischen Puderprodukt mindestens um eine der folgenden: hydrophile Glimmer, vorzugsweise Phlogopite, Bismutchloridoxid, Silikate, hydrophile Polymere, vorzugsweise Polyacrylate, Polyamide oder Polyurethane, Cellulose- oder Stärkederivate, Kaolin, Apatit-(CaOH), Zinkoxid, Titanoxid, Calciumcarbonat, Magnesiumcarbonat, Magnesiumhydrogencarbonat und/oder Mischungen derselben.

Pulverförmige Komponenten können zudem mittels verschiedener Materialien hydrophiliert werden. Unter den hydrophil beschichteten und/oder behandelten pulverförmigen Komponenten ist die Verwendung der folgenden bevorzugt: hydrophil beschichtete und/oder behandelte Polyamid-Pulver, Talk, Polyethylen-Pulver, expandierte Vinylidenchlorid-Acetonitril-Methyl(meth)acrylat-Copolymere, Polyfluoro-Pulver, Silikon-Pulver, Polyacrylat-Pulver, Polystyrol-Pulver, Pigmente und Mischungen derselben, hydrophile organische oder anorganische Pigmente.

In einer weiteren vorteilhaften Ausführungsform kann das kosmetische Puderprodukt zusätzlich mindestens eine Öl-Komponente aufweisen. Die Öl-Komponente wird hierbei als Binder eingesetzt. Das Bindersystem besteht aus meist flüssigen Bestandteilen, welche die pulverförmigen Komponenten benetzen und so dem Puderprodukt Kompaktheit und einen gewissen Zusammenhalt geben. Binder haben zudem einen großen Einfluss auf das Texturgefühl. Bei der Öl-Komponente handelt es sich vorzugsweise um eine der folgenden Substanzen oder die Öl-Komponente enthält eine oder mehrere der folgenden Substanzen:
- Poly(organo)siloxane (Silikone), vorzugsweise Methylcyclopolysiloxan, Diethylpolysiloxan, Methylphenylpolysiloxan, mit Fettsäuren modifiziertes Polysiloxan, mit Aminogruppen modifiziertes Polysiloxan, besonders bevorzugt Methylpolysiloxan und/oder Dimethylpolysiloxan,
- Esteröle, vorzugsweise Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester wie Maisöl, Avocadoöl, Kamelienöl, Olivenöl, Weizenkeimöl, Aprikosenkernöl, Sojaöl, Erdnussöl, Kakaobutter und/ oder Rizinusöl,
- Wachse, vorzugsweise tierische Wachse, pflanzliche Wachse mineralische Wachse und/oder synthetische Wachse.

Das erfindungsgemäße kosmetische Puderprodukt kann in einer weiteren Ausführungsform außerdem einen oder mehrere der folgenden Hilfsstoffe enthalten: Spurenelemente, beruhigende Stoffe, oberflächenaktive Substanzen, feuchthaltende Substanzen, rückfettende Agenzien, Fette, Alkohole, Polyole und deren toxikologisch verträglichen Ether und Ester, verzweigte und/oder unverzweigte Kohlenwasserstoffe, Antioxidantien, Vitamine, Emulgatoren, Stabilisatoren, pH-Wert-Regulatoren, Bakterizide, antimikrobielle Stoffe, Maskierungsmittel, Parfums, Silikone, Ceramide, Pflanzenextrakte, Weichmacher, Kohäsionsmittel, Schaumstabilisatoren, Verdickungsmittel, UV-Filter oder Konservierungsmittel.

In einer weiteren vorteilhaften Ausgestaltungsform handelt es sich beim kosmetischen Puderprodukt um einen Puder oder ein Rouge oder einen Lidschatten oder einen Augenbrauenpuder.

Bei dem erfindungsgemäßen kosmetischen Puderprodukt kann es sich sowohl um ein gepresstes als auch um ein loses Puderprodukt handeln.

Ebenfalls Bestandteil der vorliegenden Erfindung ist ein Verfahren zur Herstellung eines kosmetischen Puderprodukts, wobei mindestens die folgenden Verfahrensschritte nacheinander durchgeführt werden:
- Homogene Vermahlung der Puderbestandteile,
- Absenkung des pH-Wertes auf einen pH-Wert unter dem pH-Wert der Haut von pH 5,5 durch Zugabe einer Säure und homogene Vermischung,
- Zugabe von mindestens einem Farbstoff, dessen Färbung abhängig von pH-Wert und Polarität ist und homogene Vermischung.

Die Erfindung soll nachstehend durch Beispiele erläutert werden, welches sie jedoch nicht abschließend beschreibt. Die Mengenangaben erfolgen hierbei in Gewichtsprozent, jeweils bezogen auf das Gesamtgewicht der Zubereitung.

**Tabelle 1: Zusammensetzung für erfindungsgemäßes Puderprodukt**

| ***Bestandteile*** | ***Menge*** |
|---|---|
| Pulverförmige Bestandteile | 71,820 |
| Pigmente | 20,060 |
| Zitronensäure 50% | 2,000 |
| Farbstoff CI 45410 | 0,120 |
| Öle | 4,800 |
| Hilfsstoffe | 1,200 |

Zur Herstellung des erfindungsgemäßen Puderproduktes werden zunächst die pulverförmigen Bestandteile zusammengefügt und vermahlen. In einem nächsten Schritt werden die Pigmente untergehoben. Die Zitronensäure wird danach mit dem Wasser vermischt, bis diese komplett gelöst ist. Der pH-Wert der Zubereitung wird durch Zugabe der 50%igen Zitronensäure und homogene Vermischung auf einen Wert abgesenkt, der unter dem pH-Wert der Haut von pH 5,5 liegt. Vorliegend wird ein pH-Wert von pH 3,6 bis 4,1 im Endprodukt erzielt. In einem nachfolgenden Schritt wird der Farbstoff D&C Red No. 27 (CI 45410), dessen Färbung abhängig von pH-Wert und Polarität ist, zugegeben und der Ansatz homogen vermischt. Daraufhin werden die Öl-Komponenten und die Hilfsstoffe miteinander vermischt, dem Ansatz zugegeben und nochmals gleichmäßig durchmischt. Schließlich wird das Puderprodukt gepresst.

Das fertige Produkt weist eine sehr helle Farbe auf, die auf die eingesetzten Pigmente zurückzuführen ist. Je nach Auswahl der eingesetzten Pigmente, kann die Farbe im Puderprodukt nach Bedarf angepasst werden. Nach dem Applizieren auf die Haut verfärbt sich der Puder entsprechend der Farbe des Farbstoffes CI 45410. Es erfolgt ein Farbwechsel nach dem Applizieren auf die Haut von hell zu pink. Diese Farbe wird bereits nach kurzer Zeit erkennbar. Die Intensität und die Schnelligkeit der Farbentwicklung sind abhängig vom Feuchtigkeitsgehalt und pH-Wert der Haut.

Um zu untersuchen, ob sich der gepresste Puder durch Luftfeuchtigkeit verfärbt, wurde dieser einige Minuten über Wasserdampf gehalten. Nach wenigen Minuten war eine leichte pinke Verfärbung der Oberfläche zu beobachten. Nach einigen Minuten in Abwesenheit von Wasserdampf bildete sich die Verfärbung jedoch selbstständig wieder zurück. Ebenfalls wurde ein Tropfen Wasser auf den gepressten Puderstein gegeben. An der nassen Stelle war eine pinke Verfärbung zu erkennen. Diese Verfärbung bildete sich jedoch nach Trocknung des Pudersteins wieder komplett zurück. Diese Untersuchungen zeigen die Effektivität der vorliegenden Erfindung, da diese Verfärbungen reversibel sind. Das Produkt wurde zudem für 24 Stunden UV-Strahlung ausgesetzt. Zur Untersuchung der UV-Beständigkeit wurde das Puderprodukt mit einer 1,5kW Xenon-Lampe bestrahlt, wobei eine Strahlungsbelastung von 765 W/m² bei einer Wellenlänge von 320 bis 800 nm erzielt wurde. Als Negativkontrolle wurde die Probe im Dunkeln aufbewahrt. Trotz der hohen UV-Belastung war der Farbwechsel-Effekt bei Applikation auf die Haut immer noch zu beobachten.

Als Kontrolle wurde dasselbe Puderprodukt hergestellt, bei dem die pulverförmigen Puderbestandteile jedoch nicht mit Zitronensäure behandelt wurden. Die Zusammensetzung des Kontrollproduktes ist nachfolgend wiedergegeben:

**Tabelle 2: Zusammensetzung für kosmetisches Puderprodukt (Kontrolle)**

| ***Bestandteile*** | ***Menge*** |
|---|---|
| Pulverförmige Bestandteile | 73,820 |
| Pigmente | 20,060 |
| CI 45410 | 0,120 |
| Öle | 4,800 |
| Hilfsstoffe | 1,200 |

Bei dem Kontrollprodukt verfärbte sich der Ansatz direkt nach Zugabe des Farbstoffes CI 45410 pink. Es war kein Farbwechsel-Effekt mehr nach Applikation des Produktes auf die Haut zu erkennen.

1. Kosmetisches Puderprodukt enthaltend mindestens einen kosmetischen Farbstoff, dessen Färbung abhängig von pH-Wert und Polarität ist, dadurch gekennzeichnet, dass
- alle pulverförmigen Puderbestandteile des kosmetischen Puderproduktes mit einer Säure benetzt sind und
- der pH-Wert des kosmetischen Puderproduktes unter dem durchschnittlichen pH-Wert der Haut von pH 5,5 liegt.

2. Kosmetisches Puderprodukt nach einem der vorherigen Ansprüche, dadurch gekennzeichnet, dass der pH-Wert des kosmetischen Puderproduktes unter pH 5 liegt, vorzugsweise unter pH 4,5.

3. Kosmetisches Puderprodukt nach einem der vorherigen Ansprüche, dadurch gekennzeichnet, dass der kosmetische Farbstoff innerhalb des Puderproduktes keine Färbung aufweist, wobei es sich bei dem kosmetischen Farbstoff vorzugsweise um einen Xanthen-Farbstoff, besonders bevorzugt um ein Fluorescein-Derivat oder ein Rhodamin-Derivat handelt.

4. Kosmetisches Puderprodukt nach einem der vorherigen Ansprüche, dadurch gekennzeichnet, dass das Puderprodukt mindestens eine der folgenden pulverförmigen Komponenten aufweist: Füllstoffe, Pigmente, Perlmutt und Mischungen derselben.

5. Kosmetisches Puderprodukt nach einem der vorherigen Ansprüche, dadurch gekennzeichnet, dass es zusätzlich mindestens eine Öl-Komponente aufweist.

6. Kosmetisches Puderprodukt nach einem der vorangehenden Ansprüche, dadurch gekennzeichnet, dass das kosmetische Puderprodukt einen oder mehrere der folgenden Hilfsstoffe enthält: Spurenelemente, beruhigende Stoffe, oberflächenaktive Substanzen, feuchthaltende Substanzen, rückfettende Agenzien, Fette, Alkohole, Polyole und deren toxikologisch verträglichen Ether und Ester, verzweigte und/oder unverzweigte Kohlenwasserstoffe, Antioxidantien, Vitamine, Emulgatoren, Stabilisatoren, pH-Wert-Regulatoren, Bakterizide, antimikrobielle Stoffe, Maskierungsmittel, Parfums, Silikone, Ceramide, Pflanzenextrakte, Weichmacher, Kohäsionsmittel, Schaumstabilisatoren, Verdickungsmittel, UV-Filter oder Konservierungsmittel.

7. Kosmetisches Puderprodukt nach einem der vorangehenden Ansprüche, dadurch gekennzeichnet, dass es sich beim kosmetischen Puderprodukt um einen Puder oder ein Rouge oder einen Lidschatten oder einen Augenbrauenpuder handelt.

8. Verfahren zur Herstellung eines kosmetischen Puderproduktes nach einem der vorherigen Ansprüche, wobei mindestens die folgende Verfahrensschritte nacheinander durchgeführt werden:
- Homogene Vermahlung der Puderbestandteile,
- Absenkung des pH-Wertes auf einen pH-Wert unter dem pH-Wert der Haut von pH 5,5 durch Zugabe einer Säure und homogene Vermischung,
- Zugabe von mindestens einem Farbstoff, dessen Färbung abhängig von pH-Wert und Polarität ist und homogene Vermischung.

## Patentansprüche

1. Kosmetisches Puderprodukt enthaltend mindestens einen kosmetischen Farbstoff, dessen Färbung abhängig von pH-Wert und Polarität ist, **dadurch gekennzeichnet, dass**
- alle pulverförmigen Puderbestandteile des kosmetischen Puderproduktes mit einer Säure benetzt sind und
- der pH-Wert des kosmetischen Puderproduktes unter dem durchschnittlichen pH-Wert der Haut von pH 5,5 liegt, und
- der kosmetische Farbstoff innerhalb des Puderproduktes keine Färbung aufweist.

2. Kosmetisches Puderprodukt nach einem der vorherigen Ansprüche, **dadurch gekennzeichnet, dass** der pH-Wert des kosmetischen Puderproduktes unter pH 5 liegt, vorzugsweise unter pH 4,5.

3. Kosmetisches Puderprodukt nach einem der vorherigen Ansprüche, **dadurch gekennzeichnet, dass** es sich bei dem kosmetischen Farbstoff vorzugsweise um einen Xanthen-Farbstoff, besonders bevorzugt um ein Fluorescein-Derivat oder ein Rhodamin-Derivat handelt.

4. Kosmetisches Puderprodukt nach einem der vorherigen Ansprüche, **dadurch gekennzeichnet, dass** das Puderprodukt mindestens eine der folgenden pulverförmigen Komponenten aufweist: Füllstoffe, Pigmente, Perlmutt und Mischungen derselben.

5. Kosmetisches Puderprodukt nach einem der vorherigen Ansprüche, **dadurch gekennzeichnet, dass** es zusätzlich mindestens eine Öl-Komponente aufweist.

6. Kosmetisches Puderprodukt nach einem der vorangehenden Ansprüche, **dadurch gekennzeichnet, dass** das kosmetische Puderprodukt einen oder mehrere der folgenden Hilfsstoffe enthält: Spurenelemente, beruhigende Stoffe, oberflächenaktive Substanzen, feuchthaltende Substanzen, rückfettende Agenzien, Fette, Alkohole, Polyole und deren toxikologisch verträglichen Ether und Ester, verzweigte und/oder unverzweigte Kohlenwasserstoffe, Antioxidantien, Vitamine, Emulgatoren, Stabilisatoren, pH-Wert-Regulatoren, Bakterizide, antimikrobielle Stoffe, Maskierungsmittel, Parfums, Silikone, Ceramide, Pflanzenextrakte, Weichmacher, Kohäsionsmittel, Schaumstabilisatoren, Verdickungsmittel, UV-Filter oder Konservierungsmittel.

7. Kosmetisches Puderprodukt nach einem der vorangehenden Ansprüche, **dadurch gekennzeichnet, dass** es sich beim kosmetischen Puderprodukt um einen Puder oder ein Rouge oder einen Lidschatten oder einen Augenbrauenpuder handelt.

8. Verfahren zur Herstellung eines kosmetischen Puderproduktes nach einem der vorherigen Ansprüche, wobei mindestens die folgende Verfahrensschritte nacheinander durchgeführt werden:
- Homogene Vermahlung der Puderbestandteile,
- Absenkung des pH-Wertes auf einen pH-Wert unter dem pH-Wert der Haut von pH 5,5 durch Zugabe einer Säure und homogene Vermischung,
- Zugabe von mindestens einem Farbstoff, dessen Färbung abhängig von pH-Wert und Polarität ist und homogene Vermischung.
